(19) Europäisches Patentamt
European Patent Office
Office européen des brevets

(11) Veröffentlichungsnummer: **0 285 936 B1**

# (12) EUROPÄISCHE PATENTSCHRIFT

(45) Veröffentlichungstag der Patentschrift: **30.05.90**

(51) Int. Cl.[5]: **C07D 309/30,** A01N 43/16

(21) Anmeldenummer: **88104893.8**

(22) Anmeldetag: **26.03.88**

(54) **3,4-Dihydro-2H-pyrane.**

(30) Priorität: **03.04.87 DE 3711269**

(43) Veröffentlichungstag der Anmeldung:
**12.10.88 Patentblatt 88/41**

(45) Bekanntmachung des Hinweises auf die Patenterteilung:
**30.05.90 Patentblatt 90/22**

(84) Benannte Vertragsstaaten:
**BE CH DE ES FR GB GR IT LI NL**

(56) Entgegenhaltungen:
**EP-A- 0 053 756**
**WO-A-84/02910**
**CH-A- 609 053**
**US-A- 3 691 205**

**CHEMICAL AND PHARMACEUTICAL BULLETIN, Band 26, Nr. 10, 1978, Seiten 3233-3237, Tokyo, JP; T. FUJII et al.: "Synthesis of 3-tert-Butylpyridine"**
**JOURNAL OF THE CHEMICAL SOCIETY, PERKIN TRANSACTIONS I, 1979, Seiten 847 - 850, London, GB; R. MENICAGLI et al.: "Optically active heteroaromatic compounds. Part 8. A synthetic approach to (S)-2-s-Butylfuran"**
**CHEMICAL ABSTRACTS, Band 73, Nr. 19, 9. November 1970, Seite 356, Zusammenfassung Nr. 98731u, Columbus, Ohio, US; V.G. KOZYREV et al.:**

(73) Patentinhaber: **BASF Aktiengesellschaft, Carl-Bosch-Strasse 38, D-6700 Ludwigshafen(DE)**

(72) Erfinder: **Himmele, Walter, Dr., Eichenweg 14, D-6909 Walldorf(DE)**
Erfinder: **Theobald, Hans, Dr., Queichstrasse 6, D-6703 Limburgerhof(DE)**
Erfinder: **Merger, Franz, Dr., Max-Slevogt-Strasse 25, D-6710 Frankenthal(DE)**
Erfinder: **Hofmann, Ernst, Dr., Wittelsbachstrasse 84, D-6700 Ludwigshafen(DE)**
Erfinder: **Kuenast, Christoph, Dr., Salierstrasse 2, D-6701 Otterstadt(DE)**
Erfinder: **Hofmeister, Peter, Dr., Bernard-Humblot-Strasse 12, D-6730 Neustadt(DE)**

(56) Entgegenhaltungen: (Fortsetzung)
**"Aryloxydihydropyrans. IX. Diene condensation of vinyl aryl ethers with croton- and cinnamaldehydes" & KHIM. GETEROTSIKL. SOEDIN 1970, (6), 730-732a new class of positive inotropic agents"213 000**

EP 0 285 936 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Die vorliegende Erfindung betrifft neue 3,4-Dihydro-2H-pyrane der allgemeinen Formel I

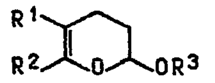

(I)

in der die Substituenten folgende Bedeutung haben:

$R^1$ $C_4$-$C_{20}$-Alkyl, $C_4$-$C_{20}$-Alkoxy-alkyl, Aryl, $C_7$-$C_{20}$-Aryl-alkyl, durch Halogen, $C_1$-$C_8$-Alkyl, $C_1$-$C_8$-Halogenalkyl, $C_1$-$C_8$-Alkoxy oder $C_1$-$C_8$-Halogenalkoxy ein- bis dreifach substituiertes Aryl oder $C_7$-$C_{20}$-Aryl-alkyl,
$R^2$ Wasserstoff oder $C_1$-$C_4$-Alkyl,
$R^3$ Aryl, durch Halogen, $C_1$-$C_8$-Alkyl, $C_1$-$C_8$-Halogenalkyl, $C_1$-$C_8$-Alkoxy oder $C_1$-$C_8$-Halogenalkoxy ein- bis dreifach substituiertes Aryl oder den Rest

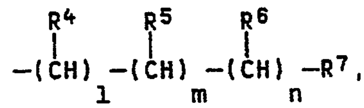

wobei
$R^4$,$R^5$,$R^6$ für Wasserstoff oder $C_1$-$C_4$-Alkyl,
$R^7$ für Phenyl, Benzoyl, durch Halogen, $C_1$-$C_8$-Alkyl, $C_1$-$C_8$-Halogenalkyl, $C_1$-$C_8$-Alkoxy oder $C_1$-$C_8$-Halogenalkoxy ein- bis dreifach substituiertes Phenyl oder Benzoyl, oder ein 5- oder 6-gliedriger Heterocyclus, enthaltend ein oder zwei Sauerstoffatome, der gegebenenfalls durch eine bis drei $C_1$-$C_8$-Alkylgruppen oder ein $C_3$-$C_6$-Spirocycloalkyl substituiert ist, und
l,m,n, für die Zahlen 0 und 1 stehen.

Außerdem betrifft die Erfindung die Herstellung der Verbindungen I, Schädlingsbekämpfungsmittel, die die Verbindungen I als Wirkstoffe enthalten, sowie ein Verfahren zur Bekämpfung von Schädlingen.

Aus Chemical Reviews 75, 651-692 (1975); den Chemical Abstracts 69, 67171z; 73, 98731u; 76, 14250n; 81, 3720t; 93, 25857t und 101, 23272h; sowie aus Khim. Geterotsikl. Soedin. 6, 730 (1970) und US 3,691,205 sind strukturell ähnliche Verbindungen bekannt, die aber nicht als Wirkstoffe für Schädlingsbekämpfungsmittel beschrieben sind.

Der Erfindung lag die Aufgabe zugrunde, neue 3,4-Dihydro-2H-pyrane I bereitzustellen.

Demgemäß wurden die eingangs definierten neuen 3,4-Dihydro-2H-pyrane I sowie Verfahren zu deren Herstellung gefunden. Überraschenderweise eignen sich die erfindungsgemäßen Verbindungen I als Schädlingsbekämpfungsmittel.

Die Verbindungen I sind nach folgenden Methoden erhältlich:

a) durch Umsetzung von Vinylethern II mit monomer vorliegenden α,β-ungesättigten Aldehyden und Ketonen III nach folgender Reaktionsgleichung:

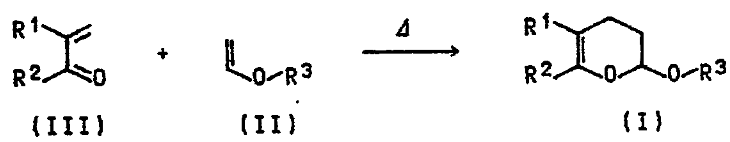


(III) (II) (I)

oder

b) durch Umsetzung von Vinylethern II mit dimer vorliegenden α,β-ungesättigten Aldehyden oder Ketonen IIIa

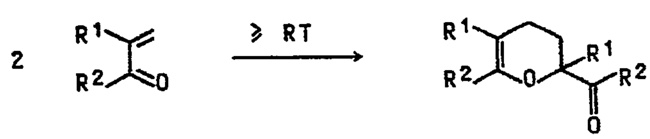


(III) monomer (IIIa) dimer

nach folgender Reaktionsgleichung

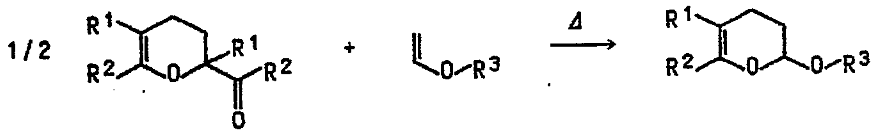

(IIIa) dimer (II) (I)

In beiden Fällen ist die Reaktionsführung gleich. Die Reaktionen a) und b) werden in Gegenwart eines Radikalinhibitors und einer basischen Verbindung bei Temperaturen von 130 bis 280°C, vorzugsweise von 160 bis 220°C, besonders bevorzugt von 180 bis 210°C, bei Drücken von 1 bis 300 bar durchgeführt. Bei diesen Reaktionen wird vorzugsweise unter Schutzgasatmosphäre gearbeitet. Als Schutzgase kommen beispielsweise Stickstoff und Argon in Betracht. Falls die Eduktkomponenten es zulassen (entsprechend hoher Siedepunkt), können die Reaktionen drucklos (bei Atmosphärendruck; ca. 1 bar) durchgeführt werden. In allen anderen Fällen empfiehlt es sich, in einem Autoklaven unter Reaktionseigendruck von > 1 bis 300 bar zu arbeiten. Die Reaktionstemperatur kann selbstverständlich durch Aufbringen eines Vordrucks auf den Autoklaven gesenkt werden. Dabei bleibt die Summe des gewählten Vordrucks und des sich beim Aufheizen zusätzlich aufbauenden Eigendrucks im Bereich bis zu 300 bar.

Als Radikalinhibitoren eignen sich aus dem Stand der Technik bekannte Polymerisationsinhibitoren wie Phenole, insbesondere substituierte Phenole, z.B. Kresole, 2,6-Di-tert.-butyl-4-methylphenol und vorzugsweise Hydrochinon oder Hydrochinonmonoalkylether wie Hydrochinonmonomethylether. Besonders bevorzugt ist Hydrochinon.

Als basische Verbindungen eignen sich beispielsweise Alkali- oder Erdalkalicarbonate oder -bicarbonate, insbesondere Natrium-, Kalium- und Calciumcarbonat, bevorzugt wird Natriumcarbonat. Die basischen Verbindungen dienen dazu, evtl. auftretende Spuren von Säure abzufangen, um eine Zersetzung der Vinylether durch Säureeinwirkung zu vermeiden. Die Bildung von Säure kann z.B. durch Abspaltungsreaktionen oder Oxidation insbesondere bei Luftzutritt oder bei Verwendung unreiner Ausgangsstoffe entstehen. Die Basenmenge ist nicht besonders kritisch, bei Verwendung reiner Ausgangsstoffe und Reaktion in einer Inertgasatmosphäre sind katalytische Mengen, z.B. 0,01 bis 0,05 mol pro Mol Vinylether ausreichend.

Im allgemeinen arbeitet man ohne Lösungs- oder Verdünnungsmittel. Man kann die Umsetzungen aber auch in Lösungs- oder Verdünnungsmitteln durchführen. Hierzu eignen sich beispielsweise aromatische Kohlenwasserstoffe wie Benzol, Toluol oder Xylol oder Ether, wie Diphenylether. Auch Gemische dieser Stoffe können als Lösungs- oder Verdünnungsmittel verwendet werden.

Zur Herstellung der erfindungsgemäßen Verbindungen I nach den voranstehend beschriebenen Methoden setzt man die Ausgangsstoffe II zu III im Molverhältnis von 0,8:1 bis 5:1, vorzugsweise 0,9:1 bis 2,5:1, besonders bevorzugt von 1:1 bis 1,5:1 ein. Entsprechendes gilt auch für die dimer vorliegenden Verbindungen IIIa.

Die Verbindungen II sind an sich bekannt oder können nach üblichen Methoden (Annalen der Chemie, Band 601, 81-138 (1956)) hergestellt werden.

Die Verbindungen III bzw. IIIa sind ebenfalls an sich bekannt oder können nach üblichen Methoden (Houben-Weyl, Methoden der organischen Chemie, Band VII/2b, 4. Auflage, 1976, Seiten 1457ff und 1482ff) erhalten werden.

Die Umsetzung von II mit III zu I kann auch unter Lewissäure-Katalyse (DE-A-21 63 515) durchgeführt werden.

Im einzelnen haben die Substituenten folgende Bedeutung:

$R^1$-$C_4$-$C_{20}$-Alkyl, bevorzugt $C_4$-$C_{12}$-Alkyl, besonders bevorzugt $C_4$-$C_8$-Alkyl, wie n-Buytl, iso-Butyl, sec.-Butyl, tert.-Butyl, n-Pentyl, iso-Pentyl, n-Hexyl, iso-Hexyl, n-Octyl, 1,3,3-Trimethyl-butyl und 2,4,4-Trimethylbutyl,

- $C_4$-$C_{20}$-Alkoxy-alkyl, bevorzugt $C_4$-$C_{12}$-Alkoxy-alkyl, besonders bevorzugt $C_4$-$C_8$-Alkoxy-alkyl, wie Methoxy-n-propyl, Methoxy-iso-propyl, n-Propoxy-methyl, iso-Propoxy-methyl, 2-Ethoxy-ethyl, tert.-Butoxy-methyl, 2-(tert.-Butoxy)-ethyl, 2-(tert.-Butoxy)-n-propyl und 2-(tert.-Butoxy)-isopropyl,

- Aryl, wie Phenyl und Naphthyl, bevorzugt Phenyl,

- $C_7$-$C_{20}$-Aryl-alkyl, bevorzugt $C_7$-$C_{12}$-Arylalkyl, besonders bevorzugt $C_7$-$C_{10}$-Aryl-alkyl, wie Benzyl, 1-Phenyl-ethyl, 2-Phenyl-ethyl, 1-Phenyl-n-propyl, 2-Phenyl-n-propyl, 2-Phenyl-iso-propyl und 3-Phenyl-n-propyl,

- durch Halogen ein- bis dreifach substituiertes Aryl, bevorzugt durch Fluor und Chlor ein- und zweifach substituiertes Phenyl, besonders bevorzugt durch Fluor oder Chlor monosubstituiertes Phenyl, wie 4-Fluorphenyl und 4-Chlorphenyl,

- durch $C_1$-$C_8$-Alkyl ein- bis dreifach substituiertes Aryl, bevorzugt durch $C_1$-$C_4$-Alkyl ein- und zweifach substituiertes Phenyl, besonders bevorzugt durch $C_1$-$C_2$-Alkyl monosubstituiertes Phenyl, wie 4-Methyl-phenyl und 4-Ethyl-phenyl,

- durch $C_1$-$C_8$-Halogenalkyl ein- bis dreifach substituiertes Aryl, bevorzugt durch $C_1$-$C_4$-Fluor- oder Chloralkyl ein- und zweifach substituiertes Phenyl, besonders bevorzugt durch $C_1$-$C_2$-Fluor- oder -Chloralkyl substituiertes Phenyl, wie 4-Trifluormethyl-phenyl und 4-Trichlormethyl-phenyl,

- durch $C_1$-$C_8$-Alkoxy ein- bis dreifach substituiertes Aryl, bevorzugt durch $C_1$-$C_4$-Alkoxy ein- und zweifach substituiertes Phenyl, besonders bevorzugt durch $C_1$-$C_2$-Alkoxy monosubstituiertes Phenyl, wie 4-Methoxy-phenyl und 4-Ethoxy-phenyl,

- durch $C_1$-$C_8$-Halogenalkoxy ein- bis dreifach substituiertes Aryl, bevorzugt durch $C_1$-$C_4$-Fluor- oder Chloralkoxy ein- und zweifach substituiertes Phenyl, besonders bevorzugt durch $C_1$-$C_2$-Fluor- oder Chloralkoxy monosubstituiertes Phenyl, wie 4-(Trifluormethoxy)-phenyl und 4-(Trichlormethoxy)-phenyl,

- durch Halogen ein- bis dreifach substituiertes $C_7$-$C_{20}$-Aryl-alkyl, bevorzugt durch Fluor und Chlor ein- und zweifach substituiertes Benzyl, besonders bevorzugt durch Fluor oder Chlor monosubstituiertes Benzyl, wie 4-Fluorbenzyl und 4-Chlorbenzyl,

- durch $C_1$-$C_8$-Alkyl ein- bis dreifach substituiertes $C_7$-$C_{20}$-Aryl-alkyl, bevorzugt durch $C_1$-$C_4$-Alkyl ein- und zweifach substituiertes Benzyl, besonders bevorzugt durch $C_1$-$C_2$-Alkyl monosubstituiertes Benzyl, wie 4-Methyl-benzyl und 4-Ethyl-benzyl,

- durch $C_1$-$C_8$-Halogenalkyl ein- bis dreifach substituiertes $C_7$-$C_{20}$-Aryl-alkyl, bevorzugt durch $C_1$-$C_4$-Fluor- oder Chloralkyl ein- und zweifach substituiertes Benzyl, besonders bevorzugt durch $C_1$-$C_2$-Fluor- oder -Chloralkyl substituiertes Benzyl, wie 4-Trifluormethyl-benzyl und 4-Trichlormethyl-benzyl,

- durch $C_1$-$C_8$-Alkoxy ein- bis dreifach substituiertes $C_7$-$C_{20}$-Aryl-alkyl, bevorzugt durch $C_1$-$C_4$-Alkoxy ein- und zweifach substituiertes Benzyl, besonders bevorzugt durch $C_1$-$C_2$-Alkoxy monosubstituiertes Benzyl, wie 4-Methoxy-benzyl und 4-Ethoxy-benzyl,

- durch $C_1$-$C_8$-Halogenalkoxy ein- bis dreifach substituiertes $C_7$-$C_{20}$-Aryl-alkyl, bevorzugt durch $C_1$-$C_4$-Fluor- oder Chloralkoxy ein- und zweifach substituiertes Benzyl, besonders bevorzugt durch $C_1$-$C_2$-Fluor- oder Chloralkoxy monosubstituiertes Benzyl, wie 4-(Trifluormethoxy)-benzyl und 4-(Trichlormethoxy)-benzyl,

wobei es sich bei den Aryl- und Arylalkylresten vorzugsweise um Phenyl- und Phenylalkylreste handelt,

$R^2$- Wasserstoff,

- $C_1$-$C_4$-Alkyl, bevorzugt $C_1$-$C_2$-Alkyl, besonders bevorzugt Methyl,

$R^3$- Aryl, wie Phenyl und Naphthyl, bevorzugt Phenyl,

- durch Halogen ein- bis dreifach substituiertes Aryl, bevorzugt durch Fluor und Chlor ein- und zweifach substituiertes Phenyl, besonders bevorzugt durch Fluor oder Chlor monosubstituiertes Phenyl, wie 2-Fluorphenyl, 4-Fluorphenyl, 2-Chlorphenyl und 4-Chlorphenyl,

- durch $C_1$-$C_8$-Alkyl ein- bis dreifach substituiertes Aryl, bevorzugt durch $C_1$-$C_4$-Alkyl ein- und zweifach substituiertes Phenyl, besonders bevorzugt durch $C_1$-$C_4$-Alkyl monosubstituiertes Phenyl, wie 4-Methyl-phenyl, 4-Ethyl-phenyl, 4-Isopropyl-phenyl, 4-sec.-Butyl-phenyl und 4-tert.-Butyl-phenyl,

- durch $C_1$-$C_8$-Halogenalkyl ein- bis dreifach substituiertes Aryl, bevorzugt durch $C_1$-$C_4$-Fluor- oder Chloralkyl ein- und zweifach substituiertes Phenyl, besonders bevorzugt durch $C_1$-$C_2$-Fluor- oder -Chloralkyl substituiertes Phenyl, wie 4-Trifluormethyl-phenyl und 4-Trichlormethyl-phenyl,

- durch $C_1$-$C_8$-Alkoxy ein- bis dreifach substituiertes Aryl, bevorzugt durch $C_1$-$C_4$-Alkoxy ein- und zweifach substituiertes Phenyl, besonders bevorzugt durch $C_1$-$C_2$-Alkoxy monosubstituiertes Phenyl, wie 4-Methoxy-phenyl und 4-Ethoxy-phenyl,

- durch $C_1$-$C_8$-Halogenalkoxy ein- bis dreifach substituiertes Aryl, bevorzugt durch $C_1$-$C_4$-Fluor- oder Chloralkoxy ein- und zweifach substituiertes Phenyl, besonders bevorzugt durch $C_1$-$C_2$-Fluor- oder Chloralkoxy monosubstituiertes Phenyl, wie 4-(Trifluormethoxy)-phenyl und 4-(Trichlormethoxy)-phenyl,

- durch Halogen und $C_1$-$C_8$-Alkyl gemeinsam zwei- und dreifach substituiertes Aryl, bevorzugt durch Fluor oder Chlor und $C_1$-$C_4$-Alkyl zweifach substituiertes Phenyl, wie 3-Methyl-4-chlorphenyl,

wobei es sich bei den Arylresten vorzugsweise um Phenylreste handelt,

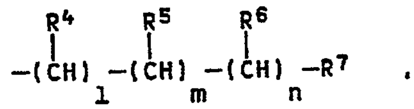

wobei
$R^4$,$R^5$,$R^6$ Wasserstoff,

- $C_1$-$C_4$-Alkyl, bevorzugt $C_1$-$C_2$-Alkyl, besonders bevorzugt Methyl,

$R^7$- Phenyl,

- Benzoyl,

- durch Halogen ein- bis dreifach substituiertes Phenyl, bevorzugt durch Fluor und Chlor ein- und zweifach substituiertes Phenyl, besonders bevorzugt durch Fluor oder Chlor monosubstituiertes Phenyl, wie 4-Fluorphenyl und 4-Chlorphenyl,

- durch $C_1$-$C_8$-Alkyl ein- bis dreifach substituiertes Phenyl, bevorzugt durch $C_1$-$C_4$-Alkyl ein- und zweifach substituiertes Phenyl, besonders bevorzugt durch $C_1$-$C_2$-Alkyl monosubstituiertes Phenyl, wie 4-Methyl-phenyl und 4-Ethyl-phenyl,

- durch $C_1$-$C_8$-Halogenalkyl ein- bis dreifach substituiertes Phenyl, bevorzugt durch $C_1$-$C_4$-Fluor- oder Chloralkyl ein- und zweifach substituiertes Phenyl, besonders bevorzugt durch $C_1$-$C_2$-Fluor- oder -Chloralkyl substituiertes Phenyl, wie 4-Trifluormethyl-phenyl und 4-Trichlormethyl-phenyl,

- durch $C_1$-$C_8$-Alkoxy ein- bis dreifach substituiertes Phenyl, bevorzugt durch $C_1$-$C_4$-Alkoxy ein- und zweifach substituiertes Phenyl, besonders bevorzugt durch $C_1$-$C_2$-Alkoxy monosubstituiertes Phenyl, wie 4-Methoxy-phenyl und 4-Ethoxy-phenyl,

- durch $C_1$-$C_8$-Halogenalkoxy ein- bis dreifach substituiertes Phenyl, bevorzugt durch $C_1$-$C_4$-Fluor- oder Chloralkoxy ein- und zweifach substituiertes Phenyl, besonders bevorzugt durch $C_1$-$C_2$-Fluor- oder Chloralkoxy monosubstituiertes Phenyl, wie 4-(Trifluormethoxy)-phenyl und 4-(Trichlormethoxy)-phenyl,

- durch Halogen ein- bis dreifach substituiertes Benzoyl, bevorzugt durch Fluor und Chlor ein- und zweifach substituiertes Benzoyl, besonders bevorzugt durch Fluor oder Chlor monosubstituiertes Benzoyl, wie 4-Fluorbenzoyl und 4-Chlorbenzoyl,

- durch $C_1$-$C_8$-Alkyl ein- bis dreifach substituiertes Benzoyl, bevorzugt durch $C_1$-$C_4$-Alkyl ein- und zweifach substituiertes Benzoyl, besonders bevorzugt durch $C_1$-$C_2$-Alkyl monosubstituiertes Benzoyl, wie 4-Methyl-benzoyl und 4-Ethyl-benzoyl,

- durch $C_1$-$C_8$-Halogenalkyl ein- bis dreifach substituiertes Benzoyl, bevorzugt durch $C_1$-$C_4$-Fluor- oder Chloralkyl ein- und zweifach substituiertes Benzoyl, besonders bevorzugt durch $C_1$-$C_2$-Fluor- oder -Chloralkyl substituiertes Benzoyl, wie 4-Trifluormethyl-benzoyl und 4-Trichlormethyl-benzoyl,

- durch $C_1$-$C_8$-Alkoxy ein- bis dreifach substituiertes Benzoyl, bevorzugt durch $C_1$-$C_4$-Alkoxy ein- und zweifach substituiertes Benzoyl, besonders bevorzugt durch $C_1$-$C_2$-Alkoxy monosubstituiertes Benzoyl, wie 4-Methoxy-benzoyl und 4-Ethoxy-benzoyl,

- durch $C_1$-$C_8$-Halogenalkoxy ein- bis dreifach substituiertes Benzoyl, bevorzugt durch $C_1$-$C_4$-Fluor- oder Chloralkoxy ein- und zweifach substituiertes Benzoyl, besonders bevorzugt durch $C_1$-$C_2$-Fluor- oder Chloralkoxy monosubstituiertes Benzoyl, wie 4-(Trifluormethoxy)-benzoyl und 4-(Trichlormethoxy)-benzoyl

- einen 5- oder 6-gliedrigen Heterocyclus, enthaltend ein oder zwei Sauerstoffatome, bevorzugt Furan-2-yl, Tetrahydrofuran-2-yl, 2H-Pyran-2-yl, 4H-Pyran-2-yl, 3,4-Dihydro-2H-pyran-2-yl und Tetrahydropyran, besonders bevorzugt 2,5-Dioxan-1-yl, 3,5-Dioxan-1-yl, 3,6-Dioxan-1-yl und 2,4-Dioxolan-1-yl.

Die 3,4-Dihydro-2H-pyrane der allgemeinen Formel I sind geeignet, Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor als Schädlingsbekämpfungsmittel eingesetzt werden.

Im Gegensatz zu den meisten bisher bekannten Wirkstoffen, die als Kontakt- oder Fraßgifte die Tiere töten, lähmen oder vertreiben, greifen die meisten der Verbindungen der Formel I in das hormonale System des tierischen Organismus ein. Bei Insekten wird beispielsweise die Umwandlung zur Imago, die Ablage von entwicklungsfähigen Eiern und die Entwicklung von abgelegten normalen Eiern gestört und dadurch die Generationsfolge unterbrochen. Für Wirbeltiere sind die erfindungsgemäßen Wirkstoffe praktisch ungiftig. Die meisten der Verbindungen der Formel I werden überdies leicht zu Stoffen abgebaut, die in der natürlichen Umgebung vorkommen und von Mikroorganismen weiter zerlegt werden.

Zu den schädlichen Insekten gehören aus der Ordnung der Schmetterlinge (Lepidoptera) beispielsweise Plutella maculipennis (Kohlschabe), Leucoptera coffeella (Kaffeemotte), Hyponomeuta malinellus (Apfelbaum gespinstmotte), Argyresthia conjugella (Apfelmotte), Sitotroga cerealella (Getreidemotte), Phthorimaea operculella (Kartoffelmotte), Capua reticulana (Apfelschalenwickler), Sparganothis pilleriana (Springwurm), Cacoecia murinana (Tannentriebwickler), Tortrix viridana (Eichenwickler), Clysia ambiguella (Heu- und Sauerwurm), Evetria buoliana (Kieferntriebwickler), Polychrosis botrana (Bekreuzter Traubenwickler), Cydia pomonella (Obstmade), Laspeyresia molesta (Pfirsichtriebbohrer), Laspeyresia funebrana (Pflaumenwickler), Ostrinia nubilalis (Maiszünsler), Loxostege sticticalis (Rübenzünsler), Ephestia kuehniella (Mehlmotte), Chilo suppressalis (Reisstengelbohrer), Galleria mellonella (Wachsmotte), Malacosoma neustria (Ringelspinner), Dendrolimus pini (Kiefernspinner), Thaumatopoea ptiyocampa (Pinienprozessionsspinner), Cheimatobia brumata (Kleiner Frostspanner), Hibernia defoliaria (Großer Frostspanner), Bupalus piniarius (Kiefernspanner), Hyphantria cunea (Weißer Bärenspinner), Agrotis segetum (Wintersaateule), Agrotis ypsilon (Ypsiloneule), Barathra brassicae (Kohleule), Cirphis unipuncta (Heerwurm), Prodenia litura (Baumwollraupe), Laphygma exigua (Rüben-Heerwurm), Panolis flammea (Forleule), Earias insulana (Baumwollkapselwurm), Plusia gamma (Gammaeule), Alabama argillacea (Baumwollblattwurm), Lymantria dispar (Schwammspinner), Lymantria monacha (Nonne), Pieris brassicae (Kohlweißling), Aporia crataegi (Baumweißling);

aus der Ordnung der Käfer (Coleoptera) beispielsweise Blitophaga undata (Schwarzer Rübenaaskäfer), Melanotus communis (Drahtwurm), Limonius californicus (Drahtwurm), Agriotes lineatus (Saatschnellkäfer), Agriotes obscurus (Humusschnellkäfer), Meligethes aeneus (Rapsglanzkäfer), Atomaria linearis (Moosknopfkäfer), Epilachna varivestis (Mexikanischer Bohnenkäfer), Phyllopertha horticola (Junikäfer), Popillia japonica (Japankäfer), Melolontha melolontha (Feldmaikäfer), Melolontha hippocastani (Waldmaikäfer), Amphimallus solstitialis (Brachkäfer), Crioceris asparagai (Spargelhähnchen), Lema melanopus (Getreidehähnchen), Leptinotarsa decemlineata (Kartoffelkäfer), Phaedon cochleariae (Meerrettich-Blattkäfer), Phyllotreta nemorum (Kohlerdfloh), Chaetocnema tibialis (Rübenflohkäfer), Phylloides chrysocephala (Rape-Flohkäfer), Diabrotica 12-puncta (Südlicher Maiswurzelwurm), Cassida nebulosa (Nebliger Schildkäfer), Bruchus lentis (Linsenkäfer), Bruchus rufimanus (Pferdebohnenkäfer), Bruchus pisorum (Erbsenkäfer), Sitona lineatus (Liniierter Blattrandkäfer), Ortiorrhynchus sulcatus (Gefurchter Lappenrüßler), Otiorrhynchus ovatus (Erdbeerwurzelrüßler), Hylobius abietis (Großer Brauner Rüsselkäfer), Byctiscus betulae (Rebenstecher), Anthonomus pomorum (Apfelblütenstecher), Anthonomus grandis (Kapselkäfer), Ceuthorrhynchus assimilis (Kohlschotenrüßler), Ceuthorrynchus napi (Großer Kohltriebrüßler), Sitophilus granaria (Kornkäfer), Anisandrus dispar (Ungleicher Holzborkenkäfer), Ips typographus (Buchdrucker), Blastophagus piniperda (Gefurchter Waldgärtner);

aus der Ordnung der Zweiflügler (Diptera) beispielsweise Lycoria pectoralis, Mayetiola destructor (Hessenfliege), Basineura brassicae (Kohlschoten-Gallmücke), Contarinia tritici (Gelbe Weizen-Gallmücke), Haplodiplosis equestris (Sattelmücke), Tipula paludosa (Wiesenschnake), Tipula oleracea (Kohlschnake), Dacus cucurbitae (Melonenfliege), Dacus oleae (Olivenfliege), Ceratitis capitata (Mittelmeerfruchtfliege), Rhagoletis cerasi (Kirschfruchtfliege), Rhagoletis pomonella (Apfelmade), Anastrepha ludens (Mexikanische Fruchtfliege), Oscinella frit (Fritfliege), Phorbia coarctata (Brachfliege), Phorbia antiqua (Zwiebelfliege), Phorbia brassicae (Kleine Kohlfliege), Pegomya hysocyami (Rübenfliege), Anopheles maculipennis, Culex pipiens, Aedes aegypti (Gelbfiebermücke), Aedes vexans, Tabanus bovinus (Rinderbremse), Tipula paludosa (Wiesenschnake), Musca domestica (Stubenfliege), Fannia canicularis (Kleine Stubenfliege), Muscina stabulans, Glossina morsitans (Tsetse-Fliege), Oestrus ovis, Chrysomya macellaria, Chrysomya hominivorax, Lucilia cuprina, Lucilia sericata, Hypoderma lineata;

aus der Ordnung der Hautflügler (Hymenoptera) beispielsweise Athalia rosae (Rübenblattwespe), Hoplocampa minuta (Pflaumensägewespe), Monomorium pharaonis (Pharaoameise), Solenopsis geminata (Feuerameise), Atta sexdens (Blattschneiderameise);

aus der Ordnung der Wanzen (Heteroptera) beispielsweise Nezara viridula (Grüne Reiswanze), Eurygaster integriceps (Asiatische Getreidewande), Blissus leucopterus (Chinch bug), Dysdercus cingulatus

(Kapok-Wanze), Dysdercus intermedius (Baumwollwanze), Piesma quadrata (Rübenwanze), Lygus pratensis (Gemeine Wiesenwanze);

aus der Ordnung der Pflanzensauger (Homoptera) beispielsweise Perkinsiella saccharicida (Zuckerrohrzikade), Nilaparvata lugens (Braune Zikade), Empoasca fabae (Kartoffelzikade), Psylla mali (Apfelblattsauger), Psylla piri (Birnblattsauger), Trialeurodes vaporariorum (Weiße Fliege), Aphis fabae (Schwarze Bohnenlaus), Aphis pomi (Grüne Apfellaus), Aphis sambuci (Holunderblattlauf), Aphidula nasturtii (Kreuzdornblattlaus), Cerosipha gossypii (Gukrenblattlauf), Sappaphis mali (Rosige Apfellaus), Sappaphis mala (Mehlige Birnblattlaus), Dysphis radicola (Mehige Apfelfaltenlaus), Brachycaudus cardui (Große Pflaumenblattlaus), Brevicoryne brassicae (Kohlblattlaus), Phorodon humuli (Hopfenblattlaus), Rhopalomyzus ascalonicus (Zwiebellaus), Myzodes persicae (Grüne Pfirsichlaus), Myzus cerasi (Schwarze Sauerkirschenlaus), Dysaulacorthum pseudosolani (Gefleckte Kartoffellaus), Acyrthosiphon onobrychis (Grüne Erbsenalaus), Macrosiphon rosae (Große Rosenblattlaus), Megoura viciae (Wickenlaus), Schizoneura lanuginosa (Birnenblattlaus), Pemphigus bursarius (Salatwurzellaus), Dreyfusia nordmannianae (Tannentrieblaus), Dreyfusia piceae (Weißtannenstammlaus), Adelges laricis (Rote Fichtengallenlaus), Viteus vitifolii (Reblaus);

aus der Ordnung der Termiten (Isoptera) beispielsweise Reticulitermes lucifugus, Calotermes flavicollis, Leucotermes flavipes, Termes natalensis;

aus der Ordnung der Geradflügler (Orthoptera) beispielsweise Forficula auricularia (Gemeiner Ohrwurm), Acheta domestica (Heimchen), Gryllotalpa gryllotalpa (Maulwurfsgrille), Tachycines asynamorus (Gewächshausschrecke), Locusta migratoria (Wanderheuschrecke), Stauronotus maroccanus (Marokkanische Wanderheuschrecke), Schistocerca peregrina (Wanderheuschrecke), Nomadacris septemfasciata (Wanderheuschrecke), Melanoplus spretus (Felsengebirgsheuschrecke), Melanoplus femur-rubrum (Rotbeinige Heuschrecke), Blatta orientalis (Küchenschabe), Blattella germanica (Deutsche Schabe), Periplaneta americana (Amerikanische Schabe), Blaberus giganteus (Riesenschabe).

Zur Klasse der Arachnoidea gehören Spinntentiere (Acarina) beispielsweise Ixodes ricinus (Holzbock), Ornithodorus moubata, Amblyomma americanum, Dermacentor silvarum, Boophilus microplus, Tetranychus telarius, Tetranychus pacificus, Paratetranychus pilosus, Bryobia praetiosa.

Zur Klasse der Nematoden zählen beispielsweise Wurzelgallennematoden, z.B. Meloidogyne incognita, Meloidogyne hapla, Meloidogyne javanica, Zysten bildende Nematoden, z.B. Globodera rostochiensis, Heterodera schatii, Heterodera avenae, Hetrodera glycinae, Hetrodera triflolii, Stock- und Blattälchen, z.B. Ditylenchus dipsaci, Ditylenchus destructor, Pratylenchus neglectus, Pratylenchus penetrans, Partylenchus goodeyi, Paratylenchus curvitatus sowie Tylenchorhynchus dubius, Tylenchorhynchus claytoni, Rotylenchus robustus, Heliocotylenchus multicinctus, Radopholus similis, Belonolaimus longicaudatus, Longidorus elongatus, Trichodorus primitivus.

Die Wirkstoffe können als solche, in Form ihrer Formulierungen oder den daraus bereiteten Anwendungsmöglichkeiten, z.B. in Form direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkali, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol, Octylphenol, Nonylphenon, Alkylphenolpolyglykolether, Tributylphenylpolyglykolether, Aklylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolylglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Beispiele für Formulierungen sind:

I. 5 Gew.-Teile der Verbindung Nr. 1 werden mit 95 Gew.-Teilen feinteiligem Kaolin innig vermischt. Man erhält auf diese Weise ein Stäubemittel, das 3 Gew.-% des Wirkstoffs enthält.

II. 30 Gew.-Teile der Verbindung Nr. 2 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

III. 10 Gew.-Teile der Verbindung Nr. 37 werden in einer Mischung gelöst, die aus 90 Gew.-Teilen Xylol, 6 Gew.-Teilen des Anlagerungsproduktes von 8 bis 10 Mol Ethylenoxid an 1 Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht.

IV. 20 Gew.-Teile der Verbindung Nr. 58 werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5 Gew.-Teilen des Anlagerungsproduktes von 40 Mol Ethylenoxid an 1 Mol Ricinusöl besteht.

V. 80 Gew.-Teile der Verbindung Nr. 61 werden mit 3 Gew.-Teilen des Natriumsalzes der Diisobutylnaphthalin-alpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten im allgemeinen zwischen 0,1 und 95 Gew.-% Wirkstoff, vorzugsweise zwischen 0,5 und 90 Gew.-%.

Die Wirkstoffkonzentrationen in den anwendungsfertigen Zubereitungen können in größeren Bereichen variiert werden.

Im allgemeinen liegen sie zwischen 0,0001 und 10 %, vorzugsweise zwischen 0,01 und 1 %. Die Wirkstoffe können auch mit gutem Erfolg im Ultra-Low-Volume-Verfahren (ULV) verwendet werden, wobei es möglich ist, Formulierungen mit mehr als 95 Gew.-% Wirkstoff oder sogar den Wirkstoff ohne Zusätze auszubringen.

Die Aufwandmenge an Wirkstoff beträgt unter Freilandbedingungen 0,2 bis 10, vorzugsweise 0,08 bis 0,8 kg/ha.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

Beispielsweise können folgende Mittel zugemischt werden: 1,2-Dibrom-3-chlorpropan, 1,3-Dichlorpropen, 1,3-Dichlorpropen + 1,2-Dichlorpropan, 1,2-Dibromethan, 2-sec.-Butyl-phenyl-N-methylcarbamat, o-Chlorphenyl-N-methylcarbamat, 3-Isopropyl-5-methylphenyl-N-methylcarbamat, o-Isopropoxyphenyl-N-methylcarbamat, 3,5-Dimethyl-4-methylmercapto-phenyl-N-methylcarbamat, 4-Dimethylamino-3,5-xylyl-N-methylcarbamat, 2-(1,3-Dioxolan-2-yl)-phenyl-N-methylcarbamat, 1-Naphthyl-N-methylcarbamat, 2,3-Dihydro-2,2-dimethyl-benzofuran-7-yl-N-methylcarbamat, 2,2-Dimethyl-1,3-benzodioxol-4-yl-N-methylcarbamat, 2-Dimethylamino-5,6-dimethyl-4-pyrimidinyl-dimethylcarbamat, 2-Methyl-2-(methylthio)-propionaldehyd-O-(methylcarbamoyl)-oxim, S-Methyl-N[(methylcarbamoyl)-oxyl]-thio-acetimidat, Methyl-N',N'-dimethyl-N-[(methylcarbamoyl)oxy]-1-thiooxamidat, N-(2-Methylchlorphenyl)-N',N'-dimethylformamidin, Tetrachlorthiophen, 1-(2,6-Difluor-benzoyl)-3-(4-chlorphenyl)-harnstoff, O,O-Dimethyl-O-(p-nitrophenyl)-phosphorthioat, O,O-Diethyl-O-(p-nitrophenyl)-phosphorthioat, O-Ethyl-O(p-nitrophenyl)-phenyl-phosphonothioat, O,O-Dimethyl-O-(3-methyl-4-nitrophenyl)-phosphorthioat, O,O-Diethyl-O-(2,4-dichlorphenyl)-phosphorthioat, O-Ethyl-O-(2,4-dichlorphenyl)-phenyl-phosphonothioat, O,O-Dimethyl-O-(2,4,5-trichlorphenyl)-phosphorthioat, O-Ethyl-O-(2,4,5-trichlorphenyl)-ethyl-phosphorthioat, O,O-Dimethyl-O-(4-brom-2,5-dichlorphenyl)-phosphorthioat, O,O-Dimethyl-O-(2,5-dichlor-4-jodphenyl)-phosphorthioat, O,O-Dimethyl-O-(3-methyl-4-methylthiophenyl)-phosphor

thioat, O-Ethyl-O-(3-methyl-4-methylthiophenyl)-isopropyl-phosphoramidat, O,O-Diethyl-O-(p-methylsulfinyl-phenyl)-phosphorthioat, O-Ethyl-S-phenyl-ethyl-phosphonodithioat, O,O-Diethyl-[2-chlor-1-(2,4-dichlorphenyl)-vinyl]-phosphat, O,O-Dimethyl-[2-chlor-1-(2,4,5-trichlorphenyl)]-vinylphosphat, O,O-Dimethyl-S-(1'-phenyl)-ethylacetat-phosphordithioat, Bis-(dimethylamino)-fluorphosphinoxid, Octamethyl-pyrophosphoramid, O,O,O,O-Tetraethyldithio-pyrophosphat, S-Chlormethyl-O,O-diethyl-phosphordithioat, O-Ethyl-S,S-dipropyl-phosphordithioat, O,O-Dimethyl-O-2,2-dichlorvinyl-phosphat, O,O-Dimethyl-1,2-dibrom-2,2-dichlorethylphosphat, O,O-Dimethyl-2,2,2-trichlor-1-hydroxy-ethylphosphonat, O,O-Dimethyl-S-[1,2-biscarbethoxy-ethyl-(1)]-phosphordithioat, O,O-Dimethyl-O-(1-methyl-2-carbmethoxy-vinyl)-phosphat, O,O-Dimethyl-S-(N-methyl-carbamoyl-methyl)-phosphordithioat, O,O-Dimethyl-S-(N-methylcarbamoyl-methyl)-phosphorthioat, O,O-Dimethyl-S-(N-methoxyethyl-carbamoylmethyl)-phosphordithioat, O,O-Dimethyl-S-(N-formyl-N-methyl-carbamoyl-methyl)-phosphordithioat, O,O-Dimethyl-O-[1-methyl-2-(methyl-carbamoyl)-vinyl]-phosphat, O,O-Dimethyl-O-[(1-methyl-2-dimethylcarbamoyl)-vinyl]-phosphat, O,O-Dimethyl-O-[(1-methyl-2-chlor-2-diethylcarbamoyl)-vinyl]-phosphat, O,O-Diethyl-S-(ethylthiomethyl)-phosphordithioat, O,O-Diethyl-S[(p-chlorphenyltthio)-methyl]-phosphordithioat, O,O-Dimethyl-S-(2-ethylthioethyl)-phosphorthioat, O,O-Dimethyl-S-(2-ethylthioethyl)-phosphordithioat, O,O-Dimethyl-S-(2-ethylsulfinyl-ethyl)-phosphorthioat, O,O-Diethyl-S-(2-ethylthioethyl)-phosphordithioat, O,O-Diethyl-S-(2-ethylsulfinyl-ethyl)-phosphorthioat, O,O-Diethyl-thiophosphoryliminophenyl)-acetonitril, O,O-Diethyl-S-(2-chlor-1-phthalimidoethyl)-phosphordithioat, O,O-Diethyl-S-[6-chlor-benzoxazolon-(2)-yl(3)]-methyldithiophosphat, O,O-Dimethyl-S-[2-methoxy-1,3,4-thiadiazol-5-[4H]-onyl-(4)-methyl]-phosphordithioat, O,O-Diethyl-O-[3,5,6-trichlor-pyridyl-(2)]-phosphorthioat, O,O-Diethyl-O-(2-pyrazinyl)-phosphorthioat, O,O-Diethyl-O-[2-isopropyl-4-methyl-pyrimidinyl(6)]-phosphorthioat, O,O-Diethyl-O-[2-(diethylamino)-6-methyl-4-pyrimidinyl]-thionophosphat, O,O-Dimethyl-S-(4-oxo-1,2,3-benzotriazin-3-[4H]-yl-methyl)-phosphordithioat, O,O-Dimethyl-S-[(4,6-diamino-1,3,5-triazin-2-yl)-methyl]-phosphordithioat, O,O-Diethyl-(1-phenyl-1,2,4-triazol-3-yl)-thionophosphat, O,S-Dimethyl-phosphor-amidothioat, O,S-Dimethyl-N-acetyl-phosphoramidothioat, alpha-Hexachlorcyclohexan, 1,1-Di-(p-methoxyphenyl)-2,2,2-trichlor-ethan, 6,7,8,9,10,10-Hexachloro-1,5,5a,6,9,9a-hexahydro-6,9-methano-2,4,3-benzodioxa-thiepin-3-oxid, Pyrethrine, DL-2-Allyl-3-methyl-cyclopenten-(2)-on-(1)-yl-(4)-DL-cis,-trans-chrysanthemat, 5-Benzyl-furyl-(3)-methyl-DL-cis,-trans-chrysanthemat, 3-Phenoxybenzyl(±)-cis,-trans-2,2-dimethyl-3-(2,2-dichlorvinyl)-cyclopropancarboxylat, alpha-Cyano-3-phenoxybenzyl(±)-cis,-trans-2,2-dimethyl-3-(2,2-dichlorvinyl)-cyclopropancarboxylat, (s)-alpha-Cyano-3-phenoxybenzyl-cis(1R,3R)-2,2-dimethyl-3-(2,2-dibromvinyl)-cyclo propancarboxylat, 3,4,5,6-Tetrahydrophthalimidoethyl-DL-cis,-trans-chrysanthemat, 2-Methyl-5-(2-propinyl)-3-furylmethyl-chrysanthemat, (alpha-Cyano-3-phenoxybenzyl)-alpha-isopropyl-4-chlorphenylacetat.

## Herstellungsbeispiele

### Beispiel 1

5-Benzyl-2-(p-chlorphenoxy)-3,4-dihydro-2H-pyran (Verbindung 1)

Unter Stickstoffatmosphäre werden 49 g 2-Benzylacrolein, 70 g p-Chlorphenylvinylether, 1 g Hydrochinon und 0,5 g Natriumcarbonat 24 Stunden bei 200°C gerührt. Anschließendes fraktioniertes Überdestillieren ergibt 78 g 5-Benzyl-2-(p-Chlorphenoxy)-3,4-dihydro-2H-pyran (Verbindung 1) in 75 %iger Ausbeute und einer Reinheit von 98 %; Sdp. 185 bis 200°C/2 mbar.

### Beispiel 2

5-(1,3,3-Trimethylbutyl)-2-(p-fluorphenoxy)-3,4-dihydro-2H-pyran (Verbindung 2)

In einem stickstoffgespülten Autoklaven werden 39 g 2-Methylen-3,5,5-trimethylhexan-1-ol, 35 g p-Fluorphenylvinylether, 0,5 g Hydrochinon und 0,5 g Natriumcarbonat 24 Stunden bei 200°C unter Eigendruck gerührt und wie üblich aufgearbeitet. Man erhält 31 g 5-(1,3,3-Trimethylbut-1-yl)-2-(p-fluorphenoxy)-3,4-dihydro-2H-pyran (Verbindung 2) in 43 %iger Ausbeute und einer Reinheit von 94 %; Sdp. 130 bis 136°C/2 mbar.

### Beispiel 3

6-Methyl-5-phenyl-2-(p-fluorphenoxy)-3,4-dihydro-2H-pyran (Verbindung 3)

In einem stickstoffgespülten Autoklaven werden 29 g 6-Methyl-5-phenyl-2-acetyl-2-phenyl-3,4-dihydro-2H-pyran, 34,5 g p-Fluorphenylvinylether, 0,5 g Hydrochinon und 0,5 g Natriumcarbonat 24 Stunden bei 190°C unter Eigendruck gerührt und wie üblich aufgearbeitet. Man erhält 29,5 g 6-Methyl-5-phenyl-2-(p-fluorphenoxy)-3,4-dihydro-2H-pyran (Verbindung 3) in 90 %iger Ausbeute, bezogen auf das eingesetzte Pyranderivat, und einer Reinheit von 98,5 %; Sdp. 127 bis 130°C/2 mbar.

Die in den nachstehenden Tabellen 1 und 2 aufgeführten Verbindungen I wurden ebenfalls auf den in den Beispielen 1 bis 3 beschriebenen Wegen erhalten.

Tabelle 1

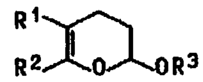

(I)

| Verbindung Nr. | $R^1$ | $R^2$ | $R^3$ | Sdp. [°C]/mbar | Fp [°C] |
|---|---|---|---|---|---|
| 4 | Benzyl | H | 2-Fluorphenyl | 152 - 160/2 | |
| 5 | 1-Phenyl-ethyl | H | 4-Chlorphenyl | 190 - 200/2 | |
| 6 | 1-Phenyl-ethyl | H | 4-Methylphenyl | 176 - 186/2 | |
| 7 | 1,3,3-Trimethylbutyl | H | 4-Chlorphenyl | 168 - 174/2 | |
| 8 | 1,3,3-Trimethylbutyl | H | 4-Methylphenyl | 157 - 166/2 | |
| 9 | Phenyl | $CH_3$ | 4-Chlorphenyl | 170 - 172/2 | |
| 10 | Phenyl | $CH_3$ | 4-Methylphenyl | 160 - 168/2 | |
| 11 | 2-tert.-Butyloxy-propyl | H | 4-Chlorphenyl | 145 - 147/2 | |
| 12 | 2-tert.-Butyloxy-propyl | H | 4-Fluorphenyl | 140 - 147/2 | |
| 13 | 2-tert.-Butyloxy-propyl | H | 4-Methylphenyl | 140/2 | |
| 14 | n-Hexyl | H | 4-Chlorphenyl | 165 - 170/2 | |
| 15 | Benzyl | H | 4-Methylphenyl | 178 - 190/2 | |
| 16 | Benzyl | H | 4-Ethylphenyl | 195 - 205/4 | |
| 17 | Benzyl | H | 2,4-Dichlorphenyl | 190 - 200/1 | |
| 18 | Benzyl | H | 3-Chlorphenyl | 180 - 185/2 | |
| 19 | Benzyl | H | 3,5-Dimethyl-4-chlorphenyl | 195 - 205/2 | |
| 20 | Benzyl | H | 4-Methoxyphenyl | 185 - 187/2 | |
| 21 | Benzyl | H | 3-Methyl-4-chlorphenyl | 186 - 190/2 | |
| 22 | Benzyl | H | 4-tert.-Butylphenyl | | 83-85 |
| 23 | Benzyl | H | 4-Fluorphenyl | 160 - 175/2 | |
| 24 | Benzyl | H | Phenyl | 160 - 170/2 | |
| 25 | Benzyl | H | 2-Chlorphenyl | 180 - 185/2 | |

Tabelle 1 (Fortsetzung)

| Verbindung Nr. | $R^1$ | $R^2$ | $R^3$ | Sdp. [°C]/mbar | Fp [°C] |
|---|---|---|---|---|---|
| 26 | 1-Phenyl-ethyl | H | 4-tert.-Butylphenyl | 190 - 200/2 | |
| 27 | 1-Phenyl-ethyl | H | 4-Fluorphenyl | 160 - 170/2 | |
| 28 | 1-Phenyl-ethyl | H | Phenyl | 162 - 170/2 | |
| 29 | 1,3,3-Trimethylbutyl | H | 4-Methoxyphenyl | 173 - 175/2 | |
| 30 | 1,3,3-Trimethylbutyl | H | Phenyl | 126/2 | |
| 31 | 2-tert.-Butyloxy-ethyl | H | 4-Methylphenyl | 140 - 142/2 | |
| 32 | 2-tert.-Butyloxy-ethyl | H | 4-Chlorphenyl | 140 - 142/3 | |
| 33 | 2-tert.-Butyloxy-ethyl | H | 4-Fluorphenyl | 135/2 | |
| 34 | 2,4,4-Trimethylbutyl | H | 4-tert.-Butylphenyl | 158 - 164/2 | |

Tabelle 2

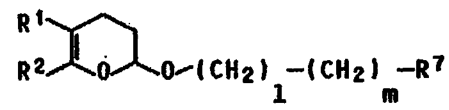

(Ia)

| Verbindung Nr. | $R^1$ | $R^2$ | $R^7$ | l | m | Sdp. [°C]/mbar |
|---|---|---|---|---|---|---|
| 35 | Benzyl | H | 1-Ethyl-4-methyl-3,5-dioxan-1-yl | 1 | 0 | 162 - 165/2 |
| 36 | Benzyl | H | 3-Methyl-2,4-dioxolan-1-yl | 1 | 0 | 160 - 168/2 |
| 37 | Benzyl | H | 3-Methyl-2,4-dioxolan-1-yl | 1 | 1 | 180 - 186/2 |
| 38 | Benzyl | H | 3,3-Dimethyl-2,4-dioxolan-1-yl | 1 | 0 | 137 - 140/2 |
| 39 | Benzyl | H | 1,3-Dimethyl-2,4-dioxolan-1-yl | 1 | 0 | 131 - 132/2 |
| 40 | Benzyl | H | 3,3-Diethyl-2,4-dioxolan-1-yl | 1 | 0 | 160 - 162/2 |
| 41 | Benzyl | H | 3,3-Spirocyclo-pentyl-2,4-dioxolan-1-yl | 1 | 0 | 177 - 180/2 |
| 42 | Benzyl | H | 3,3-Spirocyclo-hexyl-2,4-dioxolan-1-yl | 1 | 0 | 173 - 178/2 |
| 43 | 1-Phenyl-ethyl | H | 3-Methyl-2,4-dioxolan-1-yl | 1 | 0 | 175 - 180/2 |
| 44 | 1-Phenyl-ethyl | H | 3,3-Dimethyl-2,4-dioxolan-1-yl | 1 | 0 | 190/2 |
| 45 | 1-Phenyl-ethyl | H | 3-Benzyl-2,4-dioxolan-1-yl | 1 | 0 | 216 - 226/2 |
| 46 | 1-Phenyl-ethyl | H | 3-Methyl-3-(4-chlorphenyl)--2,4-dioxolan-1-yl | 1 | 0 | 210 - 226/2 |
| 47 | 1,3,3-Trimethyl-butyl | H | 3-Methyl-2,4-dioxolan-1-yl | 1 | 1 | 140 - 145/2 |
| 48 | 1,3,3-Trimethyl-butyl | H | 3,3-Dimethyl-2,4-dioxolan-1-yl | 1 | 0 | 135 - 140/2 |
| 49 | 1,3,3-Trimethyl-butyl | H | 3-Benzyl-2,4-dioxolan-1-yl | 1 | 0 | 186 - 198/2 |
| 50 | 1,3,3-Trimethyl-butyl | H | 3-Methyl-3-(4-chlorphenyl)-2,4-dioxolan-1-yl | 1 | 0 | 200 - 210/2 |
| 51 | 1,3,3-Trimethyl-butyl | H | 3,3-Spirocyclopentyl-2,4-dioxolan-1-yl | 1 | 0 | 146 - 150/2 |
| 52 | 1,3,3-Trimethyl-butyl | H | 3,3-Diethyl-2,4-dioxolan-1-yl | 1 | 0 | 140 - 150/2 |
| 53 | 1,3,3-Trimethyl-butyl | H | 3-Methyl-2,4-dioxolan-1-yl | 1 | 0 | 120 - 124/2 |

Tabelle 2 (Fortsetzung)

| Verbindung Nr. | $R^1$ | $R^2$ | $R^7$ | l | m | Sdp. [°C]/mbar |
|---|---|---|---|---|---|---|
| 54 | Phenyl | $CH_3$ | 3-Methyl-2,4-dioxolan-1-yl | 1 | 0 | 147/2 |
| 55 | Phenyl | $CH_3$ | 3-Methyl-2,4-dioxolan-1-yl | 1 | 1 | 148 - 149/2 |
| 56 | Phenyl | $CH_3$ | 3,3-Dimethyl-2,4-dioxolan-1-yl | 1 | 0 | 148/2 |
| 57 | n-Butyl | H | 3-Methyl-2,4-dioxolan-1-yl | 1 | 0 | 146 - 150/2 |
| 58 | 2-tert.-Butyloxy-ethyl | H | 3-Methyl-2,4-dioxolan-1-yl | 1 | 0 | 128 - 132/2 |
| 59 | 2-tert.-Butyloxy-ethyl | H | 3,3-Dimethyl-2,4-dioxolan-1-yl | 1 | 0 | 132 - 133/2 |
| 60 | 2-tert.-Butyloxy-ethyl | H | 3-Methyl-2,4-dioxolan-1-yl | 1 | 1 | 126 - 130/2 |
| 61 | 2-Methyl-2-tert.-Butyloxy-ethyl | H | 3-Methyl-2,4-dioxolan-1-yl | 1 | 0 | 145 - 156/4 |
| 62 | 2-tert.-Butyloxy-propyl | H | 3,3-Dimethyl-2,4-dioxolan-1-yl | 1 | 0 | 133/2 |
| 63 | 2-tert.-Butyloxy-propyl | H | 3-Methyl-2,4-dioxolan-1-yl | 1 | 1 | 140 - 143/3 |
| 64 | 1,3,3-Trimethyl-butyl | H | 1-Ethyl-4-Methyl-4-phenyl-3,5-dioxan-1-yl | 1 | 0 | 200 - 210/2 |
| 65 | 1,3,3-Trimethyl-butyl | H | 3-Methyl-2,4-dioxolan-1-yl | 1 | 0 | 120 - 126/2 |
| 66 | 1,3,3-Trimethyl-butyl | H | 3-Isopropyl-2,4-dioxolan-1-yl | 1 | 0 | 135 - 146/2 |
| 67 | 1,3,3-Trimethyl-butyl | H | 3-Isobutyl-2,4-dioxolan-1-yl | 1 | 0 | 180/4 |
| 68 | 1,3,3-Trimethyl-butyl | H | 3-Methyl-3-ethyl-2,4-dioxolan-1-yl | 1 | 0 | 122 - 124/2 |
| 69 | 1,3,3-Trimethyl-butyl | H | 3-tert.-Butyl-2,4-dioxolan-1-yl | 1 | 0 | 145 - 146/1 |
| 70 | 1,3,3-Trimethyl-butyl | H | 2,4-Dimethyl-3,6-dioxan-1-yl | 1 | 0 | 112 - 115/2 |
| 71 | 1,3,3-Trimethyl-butyl | H | 4-Isopropyl-3,5-dioxan-1-yl | 1 | 0 | 120 - 125/2 |
| 72 | 1,3,3-Trimethyl-butyl | H | 3-(2,4,4-Trimethylpentyl)-2,4-dioxolan-1-yl | 1 | 0 | 180 - 187/1 |
| 73 | 1,3,3-Trimethyl-butyl | H | 3-Methyl-2,5-dioxan-1-yl | 1 | 1 | 155/3 |

Anwendungsbeispiele

Beispiel A

Dysdercus intermedius (Baumwollwanze), Zuchtversuch

Petrischalen von 10 cm Durchmesser werden mit 1 ml der acetonischen Wirkstofflösung ausgekleidet. Nach Verdunsten des Lösungsmittels besetzt man die Schalen mit je 20 Larven des vorletzten Stadiums. Nach 24 Stunden überführt man die überlebenden Tiere in 1 l-Gläser, die 200 g sterilen Quarzsand (Korngröße 0 bis 3 mm) enthalten. Dieser Sand wurde vor Versuchsbeginn mit 25 ml der wäßrigen Wirkstoffaufbereitung angegossen. Als Futter bietet man gequollene Baumwollsamen, die wöchentlich gewechselt werden. Ebenfalls wöchentlich feuchtet man den Sand mit reinem Wasser an.

Die Versuchstemperatur beträgt 25 bis 27°C. Die Beobachtungszeit erstreckt sich bis zum Schlüpfen der Eier in den Kontrollen. Beurteilt wird:

1. wöchentlich die Mortalität
2. Häutung zum Adulten und Registrierung etwaiger Mißbildungen
3. Eiablage
4. Eischlupf

In diesem Versuch war die mortale Dosis der Verbindungen 1, 2, 7, 32, 33, 36, 54, 58, 61 und 63 kleiner oder gleich 1,0 ppm. Im allgemeinen lag die mortale Dosis der Verbindungen bei 0,4 bis 0,2 ppm, in Ausnahmefällen bei 0,004 ppm.

Beispiel B

Dysdercus intermedius (Baumwollwanze), Ovizide Wirkung

Stecketiketten werden am oberen Rand mit Klebestreifenstücken (ca. 0,8 cm) beklebt. 24 Stunden vor Versuchsbeginn werden die in einem Gefäß gesammelten Eier durch Eintauchen der Etiketten in das Gefäß an dem Klebestreifenrand befestigt. Die Eier werden für ca. 5 Sekunden in die wäßrige Wirkstofaufbereitung getaucht und auf Filterpapier abgetropft. Dabei sollen die Eier nicht auf das Papier gelegt werden. Die zurechtgeschnittenen Etiketten werden in Plastikpaletten gestellt, Klebestreifen nach oben. Eine halbe mit Wasser durchfeuchtete Watterolle wird in den Becher gelegt, um Austrocknung zu vermeiden, und die Palette wird mit einer Glasplatte abgedeckt. Nach ca. 8 Tagen wird bonitiert (bei der Kontrolle müssen die Larven geschlüpft sein).

Bei diesem Versuch war die mortale Dosis der Verbindungen 33, 39, 41, 61 und 63 kleiner oder gleich 0,1 Gew.%. Eine 80 %ige Mortalität war bei den Verbindungen 36, 37 und 58 bei einer Dosis kleiner oder gleich 0,04 Gew.% festzustellen.

**Patentansprüche**

1. 3,4-Dihydro-2H-pyrane der allgemeinen Formel I

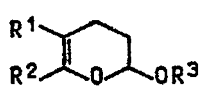

(I)

in der die Substituenten folgende Bedeutung haben:

$R^1$ $C_4$-$C_{20}$-Alkyl, $C_4$-$C_{20}$-Alkoxy-alkyl, Aryl, $C_7$-$C_{20}$-Aryl-alkyl, durch Halogen, $C_1$-$C_8$-Alkyl, $C_1$-$C_8$-Halogenalkyl, $C_1$-$C_8$-Alkoxy oder $C_1$-$C_8$-Halogenalkoxy ein- bis dreifach substituiertes Aryl oder $C_7$-$C_{20}$-Aryl-alkyl,
$R^2$ Wasserstoff oder $C_1$-$C_4$-Alkyl,
$R^3$ Aryl, durch Halogen, $C_1$-$C_8$-Alkyl, $C_1$-$C_8$-Halogenalkyl, $C_1$-$C_8$-Alkoxy oder $C_1$-$C_8$-Halogenalkoxy ein- bis dreifach substituiertes Aryl oder den Rest

$$-(\overset{R^4}{\overset{|}{CH}})_l-(\overset{R^5}{\overset{|}{CH}})_m-(\overset{R^6}{\overset{|}{CH}})_n-R^7 ,$$

wobei
$R^4$,$R^5$,$R^6$ für Wasserstoff oder $C_1$-$C_4$-Alkyl,
$R^7$ für Phenyl, Benzoyl, durch Halogen, $C_1$-$C_8$-Alkyl, $C_1$-$C_8$-Halogenalkyl, $C_1$-$C_8$-Alkoxy oder $C_1$-$C_8$-

Halogenalkoxy ein- bis dreifach substituiertes Phenyl oder Benzoyl, oder ein 5- oder 6-gliedriger Heterocyclus, enthaltend ein oder zwei Sauerstoffatome, der gegebenenfalls durch eine bis drei $C_1$-$C_8$-Alkylgruppen oder ein $C_3$-$C_6$-Spirocycloalkyl substituiert ist, und
l,m,n für die Zahlen 0 und 1 stehen.

2. 3,4-Dihydro-2H-pyrane I gemäß Anspruch 1, worin

$R^1$ $C_4$-$C_{20}$-Alkyl, $C_4$-$C_{20}$-Alkoxyalkyl oder $C_7$-$C_{20}$-Phenylalkyl,
$R^2$ Wasserstoff oder $C_1$-$C_4$-Alkyl und
$R^3$ durch Fluor oder Chlor substituiertes Phenyl bedeuten.

3. 3,4-Dihydro-2H-pyrane I gemäß Anspruch 1, worin

$R^1$ $C_4$-$C_{20}$-Alkyl, $C_4$-$C_{20}$-Alkoxyalkyl oder $C_7$-$C_{20}$-Phenylalkyl,
$R^2$ Wasserstoff oder $C_1$-$C_4$-Alkyl und
$R^3$ den Rest
$-(CH_2)_l-(CH_2)_m-R^7$
wobei
$R^7$ einen substituierten oder unsubstituierten Dioxan- oder Dioxolanrest und
l,m die Zahlen 0 oder 1 darstellen,

bedeuten.

4. Verfahren zur Herstellung von 3,4-Dihydro-2H-pyranen der allgemeinen Formel I gemäß Anspruch 1, dadurch gekennzeichnet, daß man einen Vinylether der allgemeinen Formel II

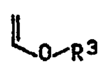 (II)

in Gegenwart eines Radikalinhibitors und einer basischen Verbindung bei Temperaturen von 130 bis 280°C und Drücken von 1 bis 300 bar in an sich bekannter Weise

a) mit einem monomer vorliegenden α,β-ungesättigten Aldehyd oder Keton III

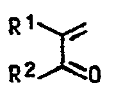 (III),

oder

b) mit einem dimer vorliegenden α,β-ungesättigten Aldehyd oder Keton IIIa

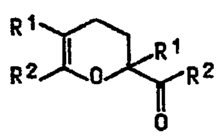 (IIIa)

umsetzt.

5. Schädlingsbekämpfungsmittel, enthaltend ein 3,4-Dihydro-2H-pyran I gemäß Anspruch 1 neben hierfür üblichen Trägerstoffen.

6. Schädlingsbekämpfungsmittel gemäß Anspruch 3, dadurch gekennzeichnet, daß es 0,1 bis 95 Gew.% eines 3,4-Dihydro-2H-pyrans I enthält.

7. Verfahren zur Bekämpfung von Schädlingen, dadurch gekennzeichnet, daß man die Schädlinge und/oder die von Schädlingen freizuhaltenden Flächen und/oder Räume mit einer für Schädlinge wirksamen Menge eines 3,4-Dihydro-2H-pyrans der Formel I gemäß Anspruch 1 behandelt.

3,4-Dihydro-2H-pyrane

Zusammenfassung

3,4-Dihydro-2H-pyrane der allgemeinen Formel I

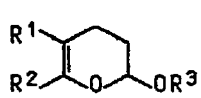 (I),

in der die Substituenten folgende Bedeutung haben:

$R^1$ $C_4$-$C_{20}$-Alkyl, $C_4$-$C_{20}$-Alkoxy-alkyl, Aryl, $C_7$-$C_{20}$-Aryl-alkyl, durch Halogen, $C_1$-$C_8$-Alkyl, $C_1$-$C_8$-

Halogenalkyl, $C_1$-$C_8$-Alkoxy oder $C_1$-$C_8$-Halogenalkoxy ein- bis dreifach substituiertes Aryl oder $C_7$-$C_{20}$-Aryl-alkyl,
$R^2$Wasserstoff oder $C_1$-$C_4$-Alkyl,
$R^3$Aryl, durch Halogen, $C_1$-$C_8$-Alkyl, $C_1$-$C_8$-Halogenalkyl, $C_1$-$C_8$-Alkoxy oder $C_1$-$C_8$-Halogenalkoxy ein- bis dreifach substituiertes Aryl oder den Rest

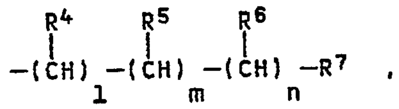

wobei
$R^4$,$R^5$,$R^6$ für Wasserstoff oder $C_1$-$C_4$-Alkyl,
$R^7$für Phenyl, Benzoyl, durch Halogen, $C_1$-$C_8$-Alkyl, $C_1$-$C_8$-Halogenalkyl, $C_1$-$C_8$-Alkoxy oder $C_1$-$C_8$-Halogenalkoxy ein- bis dreifach substituiertes Phenyl oder Benzoyl, oder ein 5- oder 6-gliedriger Heterocyclus, enthaltend ein oder zwei Sauerstoffatome, der gegebenenfalls durch eine bis drei $C_1$-$C_8$-Alkylgruppen oder ein $C_3$-$C_6$-Spirocycloalkyl substituiert ist, und
l,m,n,für die Zahlen 0 und 1 stehen
Ihre Herstellung und Verwendung zur Bekämpfung von Schädlingen.

## Claims

1. A 3,4-dihydro-2H-pyran of the formula I

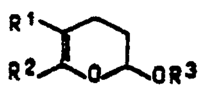

(I)

where $R^1$ is $C_4$–$C_{20}$-alkyl, $C_4$–$C_{20}$-alkoxyalkyl, aryl or $C_7$–$C_{20}$-arylalkyl, or is aryl or $C_7$–$C_{20}$-arylalkyl which has from one to three substituents selected from the group consisting of halogen, $C_1$–$C_8$-alkyl, $C_1$–$C_8$-haloalkyl, $C_1$–$C_8$-alkoxy or $C_1$–$C_8$-haloalkoxy, $R^2$ is hydrogen or $C_1$–$C_4$-alkyl, $R^3$ is aryl, or aryl having from one to three substituents selected from the group consisting of halogen, $C_1$–$C_8$-alkyl, $C_1$–$C_8$-haloalkyl, $C_1$–$C_8$-alkoxy or $C_1$–$C_8$-haloalkoxy, or the radical

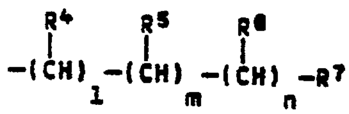

where $R^4$, $R^5$ and $R^6$ are hydrogen or $C_1$–$C_4$-alkyl, $R^7$ is phenyl or benzoyl, or phenyl or benzoyl having from one to three substituents selected from the group consisting of halogen, $C_1$–$C_8$-alkyl, $C_1$–$C_8$-haloalkyl, $C_1$–$C_8$-alkoxy, $C_1$–$C_8$-haloalkoxy, or a five- or six-membered heterocycle containing one or two oxygen atoms and which is either unsubstituted or substituted by one to three $C_1$–$C_8$-alkyl groups or one $C_3$–$C_6$-spirocycloalkyl, and l, m and n are 0 or 1.

2. A 3,4-dihydro-2H-pyran I as claimed in claim 1, where $R^1$ is $C_4$–$C_{20}$-alkyl, $C_4$–$C_{20}$-alkoxyalkyl or $C_7$–$C_{20}$-phenylalkyl, $R^2$ is hydrogen or $C_1$–$C_4$-alkyl, and $R^3$ is fluoro- or chloro-substituted phenyl.

3. A 3,4-dihydro-2H-pyran I as claimed in claim 1, where $R^1$ is $C_4$–$C_{20}$-alkyl, $C_4$–$C_{20}$-alkoxyalkyl or $C_7$–$C_{20}$-phenylalkyl, $R^2$ is hydrogen or $C_1$–$C_4$-alkyl, and $R^3$ is the radical
$-(CH_2)_l-(CH_2)m-R^7$
where $R^7$ is a substituted or unsubstituted dioxane or dioxolane radical, and l and m are 0 or 1.

4. A process for the preparation of a 3,4-dihydro-2H-pyran of the formula I as claimed in claim 1, wherein a vinyl ether of the formula II

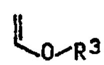

(II)

is reacted in a conventional manner in the presence of a radical inhibitor and a basic compound at from 130 to 280°C and a pressure of from 1 to 300 bar

a) with a monomeric α,β-unsaturated aldehyde or ketone III

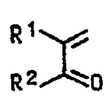

(III),

or

b) with a dimeric α,β-unsaturated aldehyde or ketone IIIa

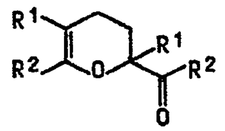 (IIIa)

5. A pesticide containing a 3,4-dihydro-2H-pyran I as claimed in claim 1, and the conventional carriers.

6. A pesticide as claimed in claim 3, containing form 0.1 to 95% by weight of a 3,4-dihydro-2H-pyran I.

7. A process for combating pests, wherein the pests and/or the areas and/or spaces to be kept free from pests, are treated with a pesticidally effective amount of a 3,4-dihydro-2H-pyran of the formula I as claimed in claim 1.

**Revendications**

1. 3,4-dihydro-2H-pyranne de formule générale I

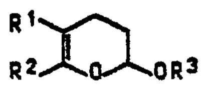 (I),

dans laquelle les substituents ont la signification suivante: $R^1$, alkyle en $C_4$–$C_{20}$, alcoxalkyle en $C_4$–$C_{20}$, aryle, arylalkyl en $C_7$–$C_{20}$, aryle ou aryl-alkyle en $C_7$–$C_{20}$ substitué une à trois fois par halogène, alkyle en $C_1$–$C_8$, halogénalkyle en $C_1$–$C_8$, alcoxy en $C_1$–$C_8$ ou halogénalcoxy en $C_1$–$C_8$, $R^2$, hydrogène ou alkyle en $C_1$–$C_4$, $R^3$, aryle, aryle substitué une à trois fois par halogène, alkyle en $C_1$–$C_8$, halogénalkyle en $C_1$–$C_8$, alcoxy en $C_1$–$C_8$ ou halogénalcoxy en $C_1$–$C_8$, ou le reste

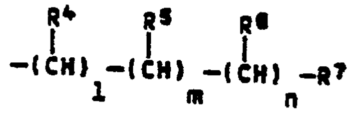

$R^4$, $R^5$, $R^6$ étant mis pour hydrogène ou alkyle en $C_1$–$C_4$, $R^7$, pour phényle, benzoyle, phényle ou benzoyle substitué une à trois fois par halogène, alkyle en $C_1$–$C_8$, halogénalkyle en $C_1$–$C_8$, alcoxy en $C_1$–$C_8$, ou halogénalcoxy en $C_1$–$C_8$, ou un hétérocycle à 5 ou 6 chaînons contenant un ou deux atomes d'oxygène, qui est éventuellement substitué par un à trois groupes alkyle en $C_1$–$C_8$ ou un spirocycloakyle en $C_3$–$C_6$, et l, m, n, pour les nombres 0 et 1.

2. 3,4-dihydro-2H-pyranne de formule générale I selon la revendication 1, où $R^1$ représente alkyle en $C_4$–$C_{20}$, alcoxalkyle en $C_4$–$C_{20}$, ou phénylalkyle en $C_7$–$C_{20}$, $R^2$, hydrogène ou alkyle en $C_1$–$C_4$ et $R^3$ phényle substitué par fluor ou chlore.

3. 3,4-dihydro-2H-pyranne de formule générale I selon la revendication 1, où $R^1$ représente alkyle en $C_4$–$C_{20}$, alcoxalkyle en $C_4$–$C_{20}$, ou alkylphényl en $C_7$–$C_{20}$, $R^2$, hydrogène ou alkyle en $C_1$–$C_4$ et $R^3$, le reste

$-(CH_2)_l-(CH_2)_m-R^7$

où $R^7$ représente un reste dioxane ou dioxolane substitué ou non substitué, et l, m, les nombres 0 ou 1.

4. Procédé de préparation de 3,4-dihydro-2H-pyranne de formule générale I selon la revendication 1, caractérisé par le fait que l'on fait réagir, de manière connue en soi, un éther vinylique de formule générale II

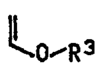 (II)

en présence d'un inhibiteur de radical et d'un composé basique, à des températures de 130 à 280°C et des pressions de 1 à 300 bar

a) avec une aldéhyde ou une cétone III, insaturée, présente à l'état monomère

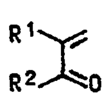 (III),

ou

b) avec une aldéhyde ou cétone IIIa, insaturée en α, β présente à l'état dimère

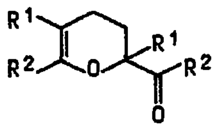

(IIIa)

5\. Agent de lutte contre les parasites, contenant une 3,4-dihydro-2H-pyranne selon la revendication 1, à côté de supports usuels.

6\. Agent de lutte contre les parasites selon la revendication 3, caractérisé par le fait qu'il contient 0,1 à 95% en poids d'une 3,4-dihydro-2H-pyranne I.

7\. Procédé de lutte contre les parasites, caractérisé par le fait qu'on traite les parasites et/ou les surfaces et/ou espaces à maintenir exempts de parasites avec une quantité efficace pour les parasites, d'une 3,4-dihydro-2H-pyranne de formule I selon la revendication 1.